# EUROPÄISCHE PATENTSCHRIFT

(11) **EP 3 094 745 B1**
(45) Veröffentlichungstag und Bekanntmachung des Hinweises auf die Patenterteilung: **30.08.2017**
(21) Anmeldenummer: 15701486.1
(22) Anmeldetag: 12.01.2015
(51) Int. Cl.: C12Q 1/68

(54) **VERFAHREN ZUR DEKONVOLUTION NUKLEINSÄURE ENTHALTENDER SUBSTANZGEMISCHE**
METHOD FOR DECONVOLUTING NUCLEIC ACID CONTAINING COMPOUND COMPOSITIONS
MÉTHODE POUR LA DECONVOLUTION DES COMPOSITIONS DE SUBSTANCES COMPRENANT DES ACIDES NUCLÉIQUES

(30) Priorität: 13.01.2014 DE 102014200446
(43) Veröffentlichungstag der Anmeldung: 23.11.2016
(73) Patentinhaber: Technische Universität Dresden, 01069 Dresden (DE)
(72) Erfinder: ZHANG, Yixin, 01219 Dresden (DE); HERRMANN, Jana, 01324 Dresden (DE); WIEDUWILD, Robert, 01069 Dresden (DE); BERTHOLD, Annett, 01309 Dresden (DE)
(74) Vertreter: Pfenning, Meinig & Partner mbB
(86) Internationale Anmeldenummer: PCT/EP2015/050414
(87) Internationale Veröffentlichungsnummer: WO 2015/104411

(56) Entgegenhaltungen:
- WO-A1-2013/137737
- Brant C. Faircloth ET AL: "Large sets of edit-metric sequence identification tags to facilitate large-scale multiplexing of reads from massively parallel sequencing", , 13. Februar 2011 (2011-02-13), XP055184176, Gefunden im Internet: URL:http://hdl.handle.net/10101/npre.2011. 5672.1 [gefunden am 2015-04-20]
- KRISHNAN A R ET AL: "Barcodes for DNA sequencing with guaranteed error correction capability", ELECTRONIC LETTERS, THE INSTITUTION OF ENGINEERING AND TECHNOLOGY, Bd. 47, Nr. 4, 17. Februar 2011 (2011-02-17), Seiten 236-237, XP006038039, ISSN: 1350-911X, DOI: 10.1049/EL:20103546
- LEONID V. BYSTRYKH ET AL: "Generalized DNA Barcode Design Based on Hamming Codes", PLOS ONE, Bd. 7, Nr. 5, 17. Mai 2012 (2012-05-17), Seite e36852, XP055182966, DOI: 10.1371/journal.pone.0036852
- TILO BUSCHMANN ET AL: "Levenshtein error-correcting barcodes for multiplexed DNA sequencing", BMC BIOINFORMATICS, BIOMED CENTRAL, LONDON, GB, Bd. 14, Nr. 1, 11. September 2013 (2013-09-11), Seite 272, XP021162969, ISSN: 1471-2105, DOI: 10.1186/1471-2105-14-272
- MARKUS LEIMBACHER ET AL: "Discovery of Small-Molecule Interleukin-2 Inhibitors from a DNA-Encoded Chemical Library", CHEMISTRY - A EUROPEAN JOURNAL, Bd. 18, Nr. 25, 18. Juni 2012 (2012-06-18) , Seiten 7729-7737, XP055133611, ISSN: 0947-6539, DOI: 10.1002/chem.201200952
- DANIEL N FRANK: "BARCRAWL and BARTAB: software tools for the design and implementation of barcoded primers for highly multiplexed DNA sequencing", BMC BIOINFORMATICS, Bd. 10, Nr. 1, 1. Januar 2009 (2009-01-01) , Seite 362, XP055003933, ISSN: 1471-2105, DOI: 10.1186/1471-2105-10-362
- Q. XU ET AL: "Design of 240,000 orthogonal 25mer DNA barcode probes", PROCEEDINGS OF THE NATIONAL ACADEMY OF SCIENCES, Bd. 106, Nr. 7, 17. Februar 2009 (2009-02-17), Seiten 2289-2294, XP055009685, ISSN: 0027-8424, DOI: 10.1073/pnas.0812506106
- BULLER F ET AL: "High-throughput sequencing for the identification of binding molecules from DNA-encoded chemical libraries", BIOORGANIC & MEDICINAL CHEMISTRY LETTERS, PERGAMON, AMSTERDAM, NL, Bd. 20, Nr. 14, 15. Juli 2010 (2010-07-15) , Seiten 4188-4192, XP027104015, ISSN: 0960-894X [gefunden am 2010-05-20]
- RALPH E. KLEINER ET AL: "Small-molecule discovery from DNA-encoded chemical libraries", CHEMICAL SOCIETY REVIEWS, Bd. 40, Nr. 12, 14. Juni 2011 (2011-06-14) , Seite 5707, XP055182892, ISSN: 0306-0012, DOI: 10.1039/c1cs15076f

## Beschreibung

Die Erfindung betrifft ein Verfahren zur Dekonvolution Nukleinsäure enthaltender Substanzgemische unter Verwendung synthetisch generierter Target-Nukleotidsequenzen.

Nukleinsäuren dienen in der Natur zur Kodierung genetischer Informationen. Verfahren zum Erkennen und Interpretieren von Nukleotidsequenzen - aus denen Nukleinsäuren gebildet sind - sind daher für viele Forschungsbereiche von großem Interesse. Mit Verfahren nach Maxam und Gilbert oder nach Sanger konnten wegweisende Grundsteine zur Sequenzierung von Nukleinsäuren gelegt werden. Auch Verfahren zur Synthese kurzer Nukleotidsequenzen (Oligonukleotidsynthese), wie beispielsweise die Phosphittriester-Methode wurden bereits etabliert und zählen zum Stand der Technik. Aufgrund dieser Fähigkeiten wurden weitere Verfahren entwickelt, die Nukleotidsequenzen, insbesondere DNA-Sequenzen als Informationsträger nutzbar machen. Eine Technologie, die sich DNA-Molekülen zur Speicherung von Informationen bedient, wird DNA-Barcoding genannt. Ziel ist es dabei, kurze DNA-Sequenzen - die sogenannten DNA-Barcodes - zu synthetisieren, um sie dann bekannten (üblicherweise größeren) Nukleotidsequenzen oder Substanzen zuzuordnen, bzw. mit der sie repräsentierenden Nukleotidsequenz oder Substanz zu koppeln. Eine Identifizierung von so präparierten Nukleotidsequenzen oder Substanzen ist dann auf einfache Weise anhand der jeweiligen DNA-Barcodes möglich, deren kurze Sequenzen in kurzer Zeit sequenziert und/oder mit entsprechenden Verfahren (PCR) amplifiziert und dadurch angereichert werden können. Aufgrund der Amplifizierbarkeit von Nukleotidsequenzen zählen Nukleinsäure basierte Verfahren im Bereich der analytischen Chemie und Biochemie zu den empfindlichsten Nachweisverfahren.

Ein weiterer Anwendungsbereich liegt in der chemischen, biologischen und medizinischen Forschung. Eine zentrale Aufgabe besteht hier in der Entdeckung molekularer Strukturen mit spezifischen Bindungsaffinitäten für Proteine. Hierzu dienen DNA-kodierte chemische Molekülbibliotheken als wirkungsvolles Werkzeug zum Aufspüren von Liganden für pharmazeutisch relevante Proteine. So können DNA-kodierte Moleküle beispielsweise durch eine affinitätsbasierte Selektion angereichert und anschließend aufgrund ihrer eindeutigen DNA-Kodierung (DNA-Barcode) entschlüsselt werden. Üblicherweise werden bei solchen Selektions-Experimenten (Screenings) DNA-kodierte Substanzgemische gewonnen. Solche Gemische enthalten üblicherweise eine Vielzahl an DNA-kodierten Substanzen.

Ungeachtet dessen wird aus Kostengründen bei der Analyse von Substanzgemischen aus Selektionsexperimenten weitläufig auf Isolierungs- bzw. Aufreinigungsschritte verzichtet. Die dabei erfassten Daten beruhen zweckmäßigerweise auf der Annahme, dass die DNA-Barcodes angereicherter Substanzen in einem solchen Gemisch mit einer höheren Wahrscheinlichkeit vorliegen und demzufolge auch mit erhöhter Wahrscheinlichkeit sequenziert werden. Diese Korrelation trifft jedoch nicht zwangsläufig zu. So kann das Ergebnis durch mehrere Faktoren, wie beispielsweise aufgrund der Transformation verschiedener Plasmide in Bakterien (in Vorbereitung zur Sanger-Sequenzierung) oder durch Annealing- und Amplifikationsprozesse auf Mikro/Nano-Strukturen (Deep Sequencing-Methoden) beeinträchtigt werden. Dieser Umstand macht einen weiteren zeitaufwendigen Verfahrensschritt erforderlich, bei dem überprüft werden muss, ob es sich bei der vermeintlich identifizierten Substanz tatsächlich um die im Gemisch angereicherte Substanz handelt.

Hinsichtlich der geringen Parallelisierbarkeit üblicher Sequenzierungsverfahren (Maxam und Gilbert oder Didesoxymethode nach Sanger) ist daher eine aufwendige Probenvorbereitung insbesondere bei Substanzgemischen, die keine signifikante Anreicherung einer gesuchten Nukleinsäure bzw. einer Nukleotidsequenz aufweisen, unvermeidbar. Ferner erfordern auch Sequenzierungsverfahren der neuen Generation, wie beispielsweise die Pyrosequenzierung eine Isolierung und Aufreinigung eines Probengemischs, bevor mit der eigentlichen Sequenzierung begonnen werden kann.

Faircloth, B.C. & Glenn, T.C. ("Large sets of edit-metric sequence identification tags to facilitate large-scale multiplexing of reads from massively parallel sequencing", Available from Nature Precedings, http://hdl.handle.net/10101/ npre.2011.5672.1; S. 1-15, 2011), Krishnan, A.R. et al. ("Barcodes for DNA sequencing with guaranteed error correction capability", Electronic Letters, The Institution of Engineering and Technology, Bd. 47, Nr. 4, S. 236-237, 2011), Bystrykh, L.V. et al. ("Generalized DNA barcode design based on hamming codes", Plos One, Bd. 7, Nr. 1, e36852, 2013) offenbaren jeweils ein Verfahren zur Generierung und Dekonvolution von Nukleotidsequenzen, wobei nach einem vorgegebenen Algorithmus voneinander verschiedene Target-Nukleotidsequenzen generiert werden, die an eine Substanz gekoppelt werden.

Es soll daher Aufgabe der Erfindung sein, ein Verfahren vorzuschlagen, mit dem individuelle Nukleotidsequenzen in Nukleinsäure enthaltenden Substanzgemischen in kurzer Zeit und kostengünstig identifiziert werden können.

Gelöst wird diese Aufgabe durch ein Verfahren gemäß Anspruch 1. Vorteilhafte Ausgestaltungen und Weiterbildungen des erfindungsgemäßen Verfahrens können mit in untergeordneten Ansprüchen bezeichneten Merkmalen realisiert werden.

Gemäß der vorliegenden Erfindung wird zur Lösung der Aufgabe ein Verfahren zur Dekonvolution Nukleinsäure enthaltender Substanzgemische vorgeschlagen, bei dem in einem ersten Schritt aus mehreren Nukleotiden (A, C, G, T/U) nach einem vorgegebenen Algorithmus mehrere sich voneinander unterscheidende Target-Nukleotidsequenzen (TNS) (A₁ - Aₙ, B₁- Bₙ, ..., Zₙ) mit N₀ - Nₙ Sequenzpositionen generiert werden. In einem weiteren Schritt wird jeweils mindestens eine der generierten TNS (A₁ - Aₙ, B₁- Bₙ, ..., Zₙ) mindestens einer Substanz oder einer Substanzkombination zugeordnet und mit dieser chemisch gekoppelt. Ferner wird bei dem erfindungsgemäßen Verfahren mindestens ein zu analysierendes Substanzgemisch mit mindestens zwei unterschiedlichen darin enthaltenen TNS und/oder TNS-gekoppelten Substanzen bereitgestellt, das nach einem Sequenzierungsverfahren sequenziert wird, wobei gleichzeitig sämtliche in dem Substanzgemisch enthaltene TNS (A₁ - Aₙ, B₁- Bₙ, ..., Zₙ) oder weitere Nukleinsäuren bzw. Nukleotidsequenzen in einem gemeinsamen Sequenzspektrum erfasst werden. Zur Erleichterung der Dekonvolution und somit zur Identifikation angereicherter TNS sollten die Sequenzspektren eines Substanzgemisches vor und nach einem Selektionsexperiment voneinander abgezogen/subtrahiert werden.

Gleichzeitig oder im Anschluss daran werden die im Sequenzspektrum überlagerten Sequenzen durch Abtasten der Sequenzpostionen N₀-Nₙ nach dem vorgegebenen Algorithmus dekonvuliert und gemäß ihrer Zuordnung als eine Substanz oder Substanzkombination identifiziert. Dabei kann beispielswiese so vorgegangen werden, dass Sequenzpositionen N₀-Nₙ, die im Sequenzspektrum eine signifikant erhöhte Signalintensität einzelner Nukleotide (A, C, G, T/U) aufweisen, nach dem vorgegebenen Algorithmus abgetastet werden.

Die Intensität eines Signals eines Nukleotids (A, C, G, T/U) an einer Sequenzposition N₀ - Nₙ entspricht der Häufigkeit eines Nukleotids (A, C, G, T/U) an der betrachteten Sequenzposition N₀ - Nₙ. Bei dem Signal kann es sich vorzugsweise um ein Lichtsignal, wie beispielsweise extern angeregte Fluoreszenz oder Chemolumineszenz handeln. Demgemäß ist die Nachweisgrenze zur Detektion eines Nukleotids (A, C, G, T/U) von einem Hintergrundrauschen bzw. von der Empfindlichkeit des verwendeten Verfahrens und/oder des Detektors abgängig.

Als signifikant ist eine Signalintensität eines Nukleotids (A, C, G, T/U) zu bewerten, wenn sie an einer Sequenzposition N₀ - Nₙ gegenüber mindestens einem Nukleotid, bevorzugt gegenüber zwei Nukleotiden, besonders bevorzugt gegenüber drei Nukleotiden um mindestens 5 %, bevorzugt um mindestens 30 % erhöht ist. Dabei können an einer Sequenzposition N₀ - Nₙ auch signifikant erhöhte Signalintensitäten für zwei oder drei Nukleotide (A, C, G, T/U) vorliegen.

Das bei der Sequenzierung aufgenommene Sequenzspektrum sollte zumindest N₀-Nₙ Sequenzpositionen zu identifizierender TNS abbilden. Besonders vorteilhaft ist es, wenn an den jeweiligen Sequenzpositionen im Sequenzspektrum eine relative Häufigkeit einzelner Nukleotide (A, C, G, T/U) dargestellt werden kann. Eine solche Häufigkeitsverteilung kann durch Vergleich der Signalintensitäten der einzelnen Nukleotide an den jeweiligen Sequenzpositionen bestimmt werden. Die Bestimmung der Häufigkeit kann auch anhand mindestens einer Standard-TNS erfolgen, die dem zu analysierenden Substanzgemisch in bekannter Konzentration zugeführt wird, bevor der Sequenzierungsschritt erfolgt.

So kann die Dekonvolution des Sequenzspektrums zusätzlich oder alternativ auch so durchgeführt werden, dass im Sequenzspektrum signifikant erhöhte Signalintensitäten einzelner Nukleotide (A, C, G, T/U) schrittweise subtrahiert werden, bis an jeder Sequenzposition N₀-Nₙ für maximal ein Nukleotid (A, C, G, T/U) die kleinste (ablesbare) Signalintensität erreicht ist, und die dabei erhaltenen Subtraktionsspektren, die jeweils mindestens eine Sequenz oder zumindest Sequenzabschnitte aufweisen, an den Sequenzpositionen N₀-Nₙ nach dem vorgegebenen Algorithmus abgetastet werden.

Der wesentliche Vorteil des erfindungsgemäßen Verfahrens liegt in der Anwendung des vorgegebenen Algorithmus, auf dessen Grundlage eine Vielzahl von TNS mit hohem Unterscheidungsgrad generiert werden können. Der hohe Unterscheidungsgrad wirkt sich insbesondere auf die Identifizierung individueller TNS günstig aus, die dadurch mit erhöhter Empfindlichkeit erfolgen kann. So kann die Identität einer TNS oder eines in Frage kommenden TNS-Kandidaten bereits durch wenige nach dem vorgebbaren Algorithmus abgetastete Sequenzpositionen einer Nukleotidfolge ermittelt werden. Auf diese Weise können auch Sequenzspektren mit mehrfach überlagernden Einzelsequenzen oder Sequenzfragmenten dekonvuliert werden, in dem nach dem vorgegebenen Algorithmus vorzugsweise Sequenzpositionen N₀ - Nₙ mit bekanntem Sequenzunterschied abgetastet werden. Eine Dekonvolution eines Nukleinsäure enthaltenden Substanzgemischs kann daher hinsichtlich einer zu identifizierenden TNS ohne Amplifikations- oder Isolationsschritte erfolgen.

Üblicherweise erfolgt die Dekodierung nach erfolgten Selektions-Experimenten durch DNA-Arrays und High-Throughput Sequencing/Deep Sequencing/Next-Generation Sequencing. Dies ist teuer und aufwendig. Durch das erfindungsgemäße Verfahren ist es möglich, nach erfolgten Selektions-Experimenten eine Nukleinsäure enthaltende Mischung, wie beispielsweise eine DNA-Mischung schnell, preiswert und auf einfache Weise, zum Beispiel mittels Sanger-Sequenzierung zu dekonvulieren.

Bei der Identifizierung angereicherter (gleicher) TNS reicht es bereits aus, dass lediglich die Sequenzpositionen des Sequenzspektrums abgetastet werden, die eine signifikant erhöhte Signalintensität für einzelne Nukleotide (A, C, G, T/U) aufweisen. Die aufgeklärten TNS dienen dann als Nachweis für ein Vorliegen der ihnen jeweils zugeordneten Substanz. Unter dem Begriff Substanz sollen hier vorzugsweise Moleküle, Molekülbestandteile und insbesondere deren funktionelle und/oder strukturelle Gruppe verstanden werden. Es kann sich bei dem Begriff Substanz je nach Anwendungsart auch um Ruß-, Tabakrauch-, Smog-, Ölrauch-, Flugasche-, Zementnebel-, Metall-, Metalloxid-, Kunststoff-, Pollen-, Bakterien- oder Viruspartikel handeln.

Eine Nukleotidbelegung für Nukleotide (A, C, G, T/U) an den Sequenzposition N₀ - Nₙ einer zu bildenden TNS (A₁ - Aₙ, B₁- Bₙ, ..., Zₙ) wird durch den vorgegebenen Algorithmus festgelegt werden. Hierzu wird für jede Sequenzposition N₀ - Nₙ eine Bedingung vorgegeben, die an eine Nukleotidbelegung mindestens einer weiteren Sequenzposition geknüpft ist. So kann bei der Bildung der TNS nach dem Algorithmus beispielsweise so vorgegangen werden, dass für jede Sequenzposition N₀₊₁ - Nₙ einer zu bildenden TNS, eine auf ein Nukleotid (A, C, G, T/U) einer vorhergehenden Sequenzposition bezogene Beschränkung für jeweils mindestens ein Nukleotid (A, C, G, T/U) vorgegeben wird.

Für eine bevorzugt einfache Identifizierung der TNS ist es vorteilhaft, wenn sich die gebildeten TNS an mindestens zwei Sequenzpositionen und/oder durch mindestens eine aus mindestens fünf Sequenzpositionen bestehende Nukleotidfolge voneinander unterscheiden. Zweckentsprechend sollten TNS, die einen Sequenzunterschied von mindestens 75%, bevorzugt mehr als 80 %, besonders bevorzugt mehr als 90 % aufweisen, jeweils Substanzen zugeordnet werden, die den größten strukturellen und/oder funktionellen Unterschied zueinander aufweisen.

Vorzugsweise sollten die TNS, die jeweils einzelne Substanzen repräsentieren, jeweils von gleicher Länge sein, d.h. jeweils die gleiche Anzahl an Sequenzpositionen N₀ - Nₙ aufweisen. So kann eine Identifizierung schon allein dadurch vereinfacht werden, dass die Dekonvolution auf eine vorgegebene Sequenzlänge beschränkt wird. Dies ermöglicht auch den direkten Vergleich an den jeweiligen Sequenzpositionen N₀ - Nₙ sich überlagernder TNS. In diesem Zusammenhang ist es ferner vorteilhaft, wenn sämtliche TNS einen gemeinsamen Sequenzabschnitt aufweisen, aufgrund dessen sie als solche identifiziert werden können. Dieser Sequenzabschnitt sollte vorzugsweise in der Start- oder Endregion einer TNS realisiert sein.

Ferner können die gebildeten TNS mindestens einen Sequenzabschnitt aufweisen, der eine Substanzgruppe, Substanzgröße, Geokoordinaten eines Expositionsortes oder ein Datum kodiert. Eigenschaften von Substanzen können auch über die Länge der TNS, d.h. über die Anzahl der Sequenzpositionen kodiert werden. Eigenschaften von Substanzen können auch in Form verschiedener Primerbindestellen kodiert werden. Beispielsweise kann eine TNS eine Geokoordinate kodieren. Mit einem weiteren Sequenzabschnitt, der als Primerbindestelle während der Sequenzierung fungiert, kann eine Substanzgruppe kodiert werden. Somit kann unter Einsatz unterschiedlicher Primer während der Sequenzierreaktion die Sequenz der jeweiligen TNS und so die Geokoordinate ermittelt werden. Anhand des jeweiligen bei der Sequenzierung eingesetzten Primers, ist es bekannt, welche Substratgruppe in diesem Fall betrachtet/berücksichtigt wird. Bei der Erstellung der Sequenz der Primerbindestelle kommt nicht der vorher beschriebene Algorithmus zum Einsatz. Stattdessen sollte diese Sequenz anhand von Parametern designt werden, die eine erfolgreiche Primerbindung/Sequenzierung erlauben. Dies können beispielsweise der G/C-Gehalt, die Primerlänge und die Primerschmelztemperatur sein.

Kombinierte Substanzen und insbesondere solche Substanzen, die infolge eines Selektions-Experiments oder Affinitäts-Experiments miteinander kombiniert worden sind, können mit entsprechend kombinierten TNS (A₁ - Aₙ, B₁ - Bₙ, ..., Zₙ) chemisch gekoppelt werden. Zweckentsprechend können TNS (A₁ - Aₙ, B₁- Bₙ, ..., Zₙ) und/oder Sequenzabschnitte von TNS (A₁ - Aₙ, B₁- Bₙ, ..., Zₙ) miteinander kombiniert werden.

Aufgrund der Vielfalt möglicher Sequenzkombinationen kann die Bildung der TNS gemäß dem vorgegebenen Algorithmus *in silico* simuliert werden. Dabei können die gebildeten TNS auch hinsichtlich möglicher Kollisionen mit bereits bekannten Nukleotidsequenzen überprüft werden.
Die chemische Synthese der TNS kann dann vorzugsweise nach der Phosphittriester-Methode erfolgen.

Da sich die Anzahl der möglichen Sequenzkombinationen nach der Anzahl der zur Verfügung stehenden Sequenzpositionen richtet, sollten TNS mit ausreichender Länge, d.h. ausreichender Anzahl an Sequenzpositionen N₀ - Nₙ gebildet werden. Dementsprechend sollten TNS mit einer Länge von mehr als fünf Sequenzpositionen gebildet bzw. synthetisiert werden.

Bei den TNS kann es sich um einzel- oder doppelsträngige RNA- oder DNA-Moleküle handeln, wobei doppelsträngige DNA-Mokelüle bevorzugt werden. Die chemische Kopplung der TNS an die sie repräsentierenden Substanzen kann bevorzugt durch kovalente Bindung erfolgen.

Im Verlauf des erfindungsgemäßen Verfahrens kann mindestens ein Verfahrensschritt zur Selektion von mindestens einer TNS (A₁ - Aₙ, B₁- Bₙ, ..., Zₙ) oder mindestens einer TNS- gekoppelten Substanz vorgesehen sein. So kann ein erster Selektionsschritt beispielsweise nach der Bildung/Synthese der TNS durchgeführt werden, um fehlerhafte TNS zu entfernen. Ein weiterer Selektionsschritt kann nach Kopplung der TNS an ihre zugeordneten Substanzen erfolgen, so dass fehlerhaft angekoppelte Substanzen ermittelt und somit die Qualität der Bibliothek evaluiert werden kann. Dabei kann jeweils so vorgegangen werden, dass die zu selektierende Komponente (Substanz oder TNS), die sich in einer flüssigen mobilen Phase befindet, an einer stationären Phase, in der entsprechende Bindungspartner bzw. Konjugate der zu selektierenden Komponente immobilisiert sind, gebunden wird. So kann jeweils ein zu analysierendes Substanzgemisch aus einer flüssigen mobilen Phase und/oder aus einer flüssigen Elution einer stationären Phase bereitgestellt werden.

Alternativ oder zusätzlich kann der Anteil an unerwünschten oder fehlerhaft mit TNS-gekoppelten Substanzen in einem weiteren Selektionsschritt ermittelt werden. Dabei werden die unerwünschten Substanzen durch eine Abbruchreaktion unter Einsatz eines Terminationsreagenz, das die gleiche reaktive Gruppe enthält, wie die Substanzbausteine des vorhergehenden Reaktionsschrittes. Somit kann das Terminationsreagenz mit noch unreagierten Vorläufermolekülen reagieren und diese markieren, so dass das Terminationsreagenz auch als Markersubstanz bezeichnet werden kann. Eine Markersubstanz kann, beispielsweise um eine Ankopplung an eine stationäre Phase zu ermöglichen, eine RNA- oder DNA-Sequenz, ein Biotin- oder Streptavidin/Avidin- Molekül und/oder ein Azid/Alkin aufweisen.

Das so präparierte Substanzgemisch kann dann in Form einer mobilen Phase mit einer stationären Phase, in der entsprechende Fänger- Domänen, Konjugate, RNA- und/oder DNA-Sequenzen zur Kopplung der Markersubstanz immobilisiert sind, in Verbindung gebracht werden, wobei die unerwünschten Substanzen an der stationären Phase gebunden, ermittelt und/oder quantifiziert werden können. Als Marker-Fängersystem kommen zum beispielsweise die Biotin-Streptavidin/Avidin-, die DNA/DNA-, die RNA/RNA- oder die Azid-Alkyl-Huisgen Klick-Reaktion in Frage.

Die zuvor beschriebenen Selektionsschritte können insbesondere im Anschluss an eine erste Sequenzierung eines zu analysierenden Substanzgemischs erfolgen. Hierzu können bereits in einem ersten Sequenzierungsschritt identifizierte TNS oder TNS-gekoppelte Substanzen aus dem zu analysierenden Substanzgemisch entfernt werden. Ferner kann dadurch auch eine Isolation von TNS oder TNS-gekoppelten Substanzen erreicht werden. Damit können weitere TNS durch erneute Sequenzierung des verbleibenden Substanzgemisches identifiziert werden, die in einer geringeren Menge vorliegen. Ein oder mehrere Selektionsschritt(e) zur Selektion mindestens einer TNS oder TNS-gekoppelten Substanzen kann/können daher zur Bereitstellung des zu analysierenden Substanzgemischs vorgesehen sein.

Hinsichtlich des zu verwendenden Sequenzierungsverfahrens - zur Sequenzierung eines zu analysierenden Substanzgemischs - wird keine Beschränkung vorgegeben. Bevorzugt kann eine Sequenzierung nach Sanger unter Verwendung fluoreszenzmarkierter Didesoxy-Nukleotide, mindestens einer Polymerase und mindestens eines Primers, der mit einem Sequenzabschnitt mindestens einer TNS komplementär ist, durchgeführt werden.

Im Folgenden wird das erfindungsgemäße Verfahren anhand von Ausführungs- und Anwendungsbeispielen in Verbindung mit Figuren näher erläutert.

Dabei zeigen:
- Figur 1:: ein Beispiel eines Algorithmus zur *in silico* Generierung unterschiedlicherTarget-Nukleotidsequenzen (TNS).
- Figur 2:: ein Beispiel eines Selektionsschritts zur Selektion von TNS
- Figur 3:: ein Beispiel einer Dekonvolution eines Sequenzspektrums nach dem vorgegebenen Algorithmus
- Figur 4a/b:: eine Prinzipskizze eines Anwendungsbeispiels des erfindungsgemäßen Verfahrens

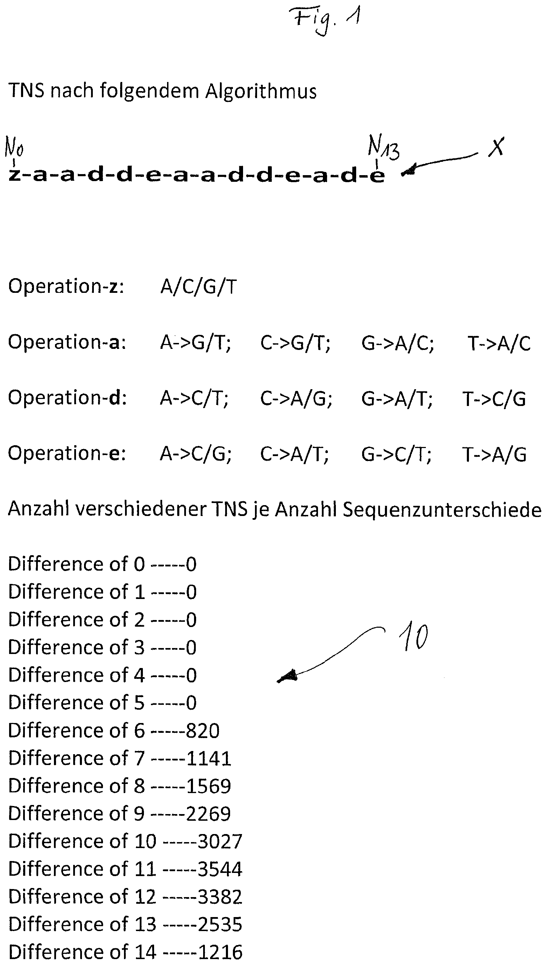

In Figur 1 ist die Generierung von verschiedenen Target-Nukleotidsequenzen (TNS) an einem Beispiel dargestellt. Die Generierung der TNS erfolgt nach dem Algorithmus X *in silico.* Zunächst wird die Länge der zu bildenden TNS durch Vorgabe der Sequenzpositionen festgelegt. Im vorliegenden Fall beträgt die Sequenzlänge 14 Positionen N₀-N₁₃, wobei für jede Sequenzposition N₀-N₁₃ eine Operation z, a, d oder e vorgegeben wird. An der Sequenzposition N₀ wird mit der Operation z keine Beschränkungen für ein Nukleotid A, C, G, T vorgegeben. Für jede weitere Sequenzposition N₁- N₁₃ wird jeweils nach den Operationen a, d, oder e eine auf ein Nukleotid A, C, G, T einer vorhergehenden Sequenzposition bezogene Beschränkung für jeweils zwei Nukleotide A, C, G oder T vorgegeben. Der Algorithmus X weist die Form z-a-a-d-d-e-a-a-d-d-e-a-d-e auf. Eine Auswahl so gebildeter TNS kann der im Anhang angefügten Code-Tabelle entnommen werden (siehe S. 15 bis 18 in der Beschreibung). Wie aus der Tabelle mit dem Bezugszeichen 10 in Figur 1 ersehen werden kann, weist ein beliebig ausgewähltes (generiertes) TNS-Paar mindestens sechs Sequenzunterschiede auf.

Nicht dargestellt, besteht die Möglichkeit zur Erweiterung/Weiterbildung der so gebildeten TNS mit weiteren Sequenzpositionen, durch die weitere Informationen hinterlegt (kodiert) werden können. So können zusätzliche Sequenzpositionen oder Sequenzabschnitte vorgesehen sein, die jeweils Eigenschaften von zu koppelnden Substanzen, wie beispielsweise eine Substanzgruppe, Substanzgröße, Geokoordinaten eines Expositionsortes oder ein Datum kodieren. Ferner können auch ergänzende Sequenzpositionen vorgesehen sein, anhand deren die TNS als solche identifiziert werden können. Dabei sollten gattungsgleiche TNS gleiche Sequenzlängen aufweisen.

Im Anschluss daran wird eine *in vitro-*Synthese der *in silico* generierten TNS mittels der Phosphittriester-Methode durchgeführt.

Die chemische Kopplung der synthetisierten TNS an die sie repräsentierenden Substanz kann mittels der Ausbildung einer Amid-Bindung unter Zuhilfenahme von Peptidkopplungsreagenzien erfolgen.

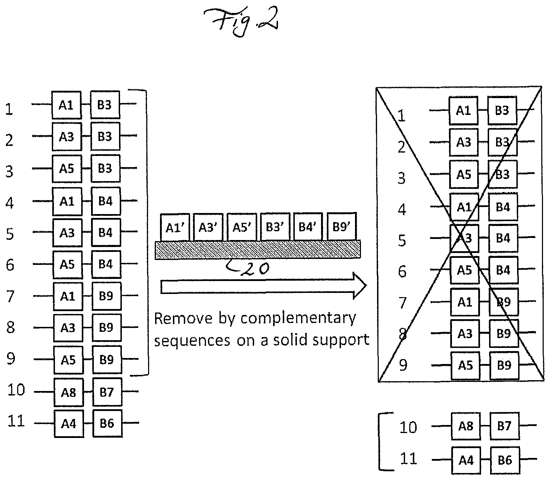

Die Fig. 2 zeigt ein Beispiel eines Selektionsschritts bei dem bei einer ersten Sequenzierung TNS als angereichert identifiziert und aus einem Gemisch entfernt werden können. Dadurch wird es ermöglicht, dass weitere TNS durch Sequenzierung identifiziert werden können, die aufgrund ihrer geringeren Anzahl im Substanzgemisch (und damit verbundenen geringeren Signalintensität) bei der ersten Sequenzierung durch die Signalintensität der häufig auftretenden TNS überdeckt worden sind. Dadurch können nicht nur die am stärksten angereicherten, sondern auch weitere, in geringerem Maße angereicherte TNS identifiziert werden.

Im vorliegenden Beispiel ist auf der linken Seite ein TNS-Gemisch mit 11 Positionen dargestellt, wobei jede Position eine kombinierte TNS repräsentiert. Die Großbuchstaben stellen dabei jeweils eine einzelne TNS dar. Mit dem Bezugszeichen 20 ist eine stationäre Phase gekennzeichnet, an der Nukleotidsequenzen A1', A3', A5', B3', B4' und B9' immobilisiert sind, die gegenüber den TNS A1, A3, A5, B3, B4 und B9 komplementär sind und somit eine Anbindung der genannten TNS ermöglichen. Wird das die Positionen 1 bis 11 enthaltende TNS-Gemisch als mobile Phase mit der stationären Phase 20 in Verbindung gebracht, werden die Positionen 1 bis 9 an der stationären Phase 20 gebunden und aus der mobilen Phase entfernt. Das aus diesem Selektionsexperiment hervorgehende TNS-Gemisch (rechte Seite) enthält dann nur noch die TNS der Positionen 10 und 11.

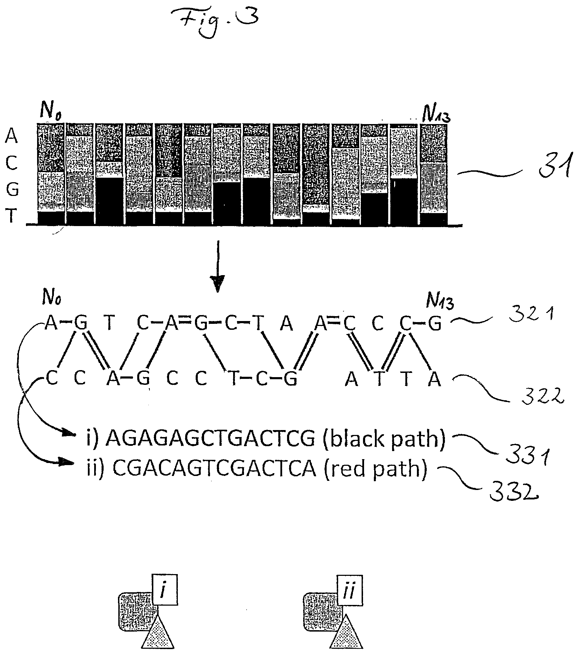

Die Figur 3 zeigt ein Ablaufschema eines Beispiels zur Dekonvolution eines Sequenzspektrums 31, das bei einer Sequenzierung eines Nukleinsäure enthaltenden Substanzgemischs aufgenommen worden ist. Im vorliegenden Beispiel wurde die Sequenzierung nach Sanger unter Verwendung fluoreszenzmarkierter Didesoxy-Nukleotide durchgeführt. Aufgrund der Signalintensität der Didesoxy-Nukleotide konnte an den jeweiligen Sequenzpositionen N₀-N₁₃ im Sequenzspektrum 31 eine relative Häufigkeit für die Nukleotide A, G, C, T bestimmt werden, die in Form von Balken dargestellt ist. Aufgrund der Häufigkeitsverteilung der jeweiligen Nukleotide an den Sequenzpositionen N₀-N₁₃ können aus dem Sequenzspektrum 31 verschiedene TNS-Kandidaten, die für eine Identifizierung in Frage kommen, abgeleitet werden. Im vorliegenden Beispiel wurden die TNS-Kandidaten 321 und 322 ermittelt. Diese werden anhand des vorgegebenen Algorithmus X mit der Operationsfolge
z-a-a-d-d-e-a-a-d-d-e-a-d-e
abgetastet und dekonvuliert. Aufgrund der Dekonvolution können zwei TNS 331 und 332 identifiziert werden, wobei die TNS 331 die Substanz i und die TNS 332 die Substanz ii repräsentieren.

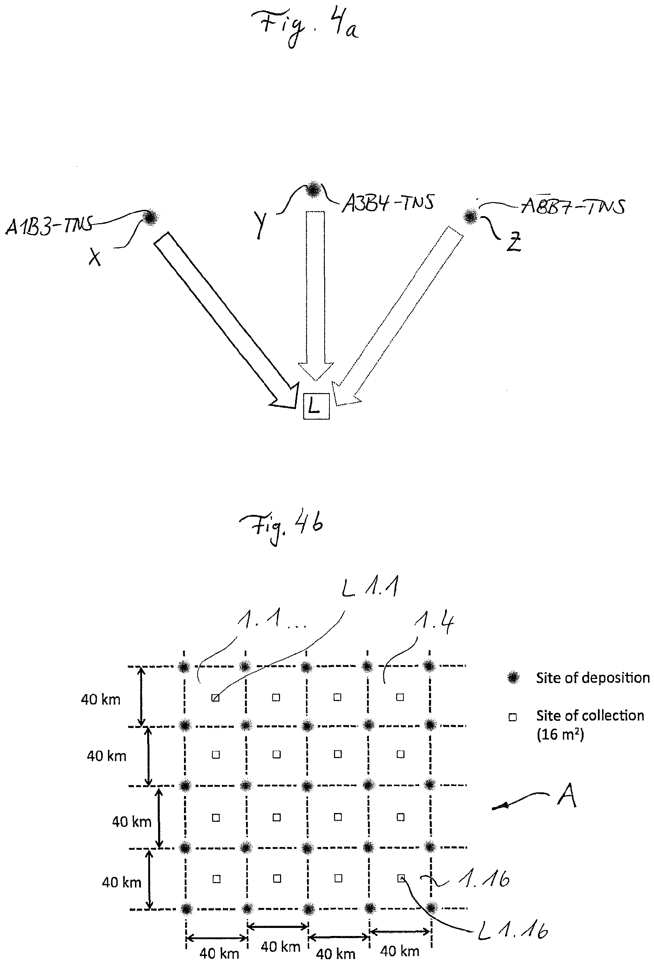

Die Fig. 4a zeigt eine Prinzipskizze eines Anwendungsbeispiels TNSgekoppelter Substanzen. Gemäß dem Ausführungsbeispiel soll die Ausbreitung von TNS-gekoppelten bzw. TNS-markierten Partikel in der Umwelt nachvollzogen werden. Hierzu werden mit TNS-A1B3 gekoppelte Partikel an der Position X, Partikel mit der TNS-Kopplung A3B4 an der Position Y und Partikel mit der TNS-Kopplung A8B7 an der Position Z ausgesetzt. Bei den Partikeln handelt es sich beispielsweise um Schadstoffpartikel mit einer Größe im Bereich von 10 nm bis 100 µm. Die jeweiligen Positionen X, Y oder Z, an denen die Partikel ausgesetzt werden, sind in einem separaten Sequenzabschnitt der jeweiligen TNS kodiert. Ferner können die TNS auch einen separaten Sequenzabschnitt aufweisen, der das Expositionsdatum (an der jeweiligen Position X, Y, Z) der Partikel kodiert. Eine Probenentnahme kann beispielsweise an einer Position L erfolgen. Dies kann beispielsweise mit einem entsprechenden Luftfilter realisiert werden. Die mit dem Luftfilter erfassten Partikel werden dann in einer Flüssigkeit dispergiert. Das so bereitgestellte Nukleinsäure enthaltende Substanzgemisch kann nachfolgend einer Sequenzierung unterzogen werden. Dabei kann es vorteilhaft sein, das zu analysierende Nukleinsäure enthaltende Substanzgemisch vor der Sequenzierung einer Amplifikation mittels PCR zu unterziehen, wobei entsprechende Primer für TNS A1B3, TNS A3B4 und TNS A8B7 eingesetzt werden. Auf diese Weise kann das Vorhandensein von Partikeln TNS A1B3, TNS A3B4 und TNS A8B7 markierten Partikeln an der Position L ermittelt werden, wodurch eine Migration der Partikel von den Positionen X, Y und Z nachvollzogen werden kann.

Die Figur 4b zeigt ein Beispiel eines Schemas, nach dem TNS markierte Partikel ausgesetzt und eingefangen werden können. Hierzu ist eine aus 16 Kleinquadraten 1.1-1.16 gebildete Fläche A dargestellt. Die Kantenlänge der Kleinquadrate beträgt z.B. jeweils 40 km, so dass eine Fläche A in diesem Fall eine Ausdehnung von 1600 km² aufweist. Die kreisförmig dargestellten Punkte an den Ecken der Kleinquadrate 1.1-1.16 kennzeichnen jeweils einen Standort, an dem TNS-markierte Partikel ausgesetzt werden, wobei jeweils vorzugsweise mittig in den Kleinquadraten eine Fläche L1.1- L1.16 von mindestens 16 m² vorgesehen ist, um TNS-markierte Partikel einzufangen bzw. zu detektieren.

Mit den erfassen TNS-markierten Partikeln kann eine lokale Zuordnung der jeweiligen Position, an der ein oder mehrere TNS-markierte Partikel lokalisiert und analysiert worden sind, erreicht werden. Dies ist insbesondere Vorteilhaft, wenn eine bestimmte lokale Verteilung von TNS-markierten Partikeln, die durch äußere Einflüsse von einer Position zu anderen Positionen bewegt worden sind, erfasst werden soll.

Das beschriebene Anwendungsbeispiel ist auch auf aquatische Systeme adaptierbar.

Ein weiterer Anwendungsbereich liegt in der chemischen, biologischen und medizinischen Forschung. So können TNS beispielsweise eingesetzt werden, um molekulare Strukturen mit spezifischen Bindungsaffinitäten für Proteine zu identifizieren. Ferner können auch TNS- bzw. DNA-kodierte chemische Molekülbibliotheken als wirkungsvolles Werkzeug zum Aufspüren von Liganden für pharmazeutisch relevante Proteine eingesetzt werden. So können TNS- bzw. DNA-kodierte Moleküle beispielsweise durch eine affinitätsbasierte Selektion angereichert und anschließend aufgrund ihrer eindeutigen TNS- bzw. DNA-Kodierung entschlüsselt werden. Die bei solchen Selektions-Experimenten gewonnenen TNS- bzw. DNA-kodierten Substanzgemische können dann nach dem erfindungsgemäßen Verfahren auf einfache Weise dekonvuliert werden, ohne dass eine Isolierung/Aufreinigung oder Amplifikation des Gemisch erforderlich ist.

### Anhang

**Code -Tabelle**

| Anzahl (= SEQ ID NO.) | TNS-Code | Round Nr. |
|---|---|---|
| 1 | GCGATGAGACATGT | 0 |
| 2 | ATCATATACGTATA | 1 |
| 3 | TAGACATCATAGAG | 2 |
| 4 | TATGTGCTCGCGAG | 3 |
| 5 | AGATGCTATGTCAC | 4 |
| 6 | TCTCGTAGTCTCGT | 5 |
| 7 | TCGATGATCACTCT | 6 |
| 8 | GCTCAGCTGTGCAG | 7 |
| 9 | CTCGAGATCGCTGC | 8 |
| 10 | CGCACTAGATGCGT | 9 |
| 11 | GCTCGCGCGAGCAC | 10 |
| 12 | AGCGTGAGTCTCAG | 11 |
| 13 | CTCGACTATCAGAC | 12 |
| 14 | CGATGCTCATAGTA | 14 |
| 15 | ATCGACGCATGCAG | 15 |
| 16 | GAGTGCGATCAGAG | 17 |
| 17 | CTACACTCACTACA | 18 |
| 18 | TATCGTCGATGATA | 20 |
| 19 | TCTGTATATCTCAC | 21 |
| 20 | GATCACTCGTATCA | 23 |
| 21 | AGCATAGCGACGTA | 24 |
| 22 | TCTGAGCGATAGTA | 25 |
| 23 | ATATCTCTGACGTG | 27 |
| 24 | GAGTCTAGACTCAG | 29 |
| 25 | GAGTGTAGTGTACA | 30 |
| 26 | GCTGTGCTGAGATA | 31 |
| 27 | CTACGTATGTATCT | 32 |
| 28 | TATCGCGACGTATA | 34 |
| 29 | GAGACTCGTGCGTG | 36 |
| 30 | TATCGCTCACAGAC | 37 |
| 31 | TATCGCTACAGCGT | 40 |
| 32 | CTCACTCTCAGCAG | 41 |
| 33 | GATCAGCTCACTGT | 47 |
| 34 | AGCGTGCTGTATGT | 50 |
| 35 | CGACGCGCGACGAG | 52 |
| 36 | TCGATAGACAGATG | 53 |
| 37 | GCGATGCTGTATCA | 54 |
| 38 | CTACGCGATGCTGC | 55 |
| 39 | CGCACAGCACAGTG | 56 |
| 40 | GCGTCAGATGCTCA | 57 |
| 41 | TAGTCTCGATGCGC | 59 |
| 42 | ATCACAGCATGACA | 60 |
| 43 | CTATGCTACACGAC | 68 |
| 44 | CGCACAGATGTCGT | 72 |
| 45 | AGACAGATGAGACT | 74 |
| 46 | ATATCATCGTATGT | 79 |
| 47 | GATGTAGCACTACT | 84 |
| 48 | TATGTATCGACTCT | 88 |
| 49 | CGACAGAGACAGTG | 92 |
| 50 | CTCGTAGATCATGT | 99 |
| 51 | CGACGTCTCGTCGT | 100 |
| 52 | TAGTGCGACGCTCT | 105 |
| 53 | GAGACAGACACTGT | 108 |
| 54 | GCGACTCGATGACA | 109 |
| 55 | AGACACGCGTGATA | 114 |
| 56 | ATCATATCACTCAG | 118 |
| 57 | GATGTATCATATGC | 122 |
| 58 | GCTCGTCGTCAGTA | 124 |
| 59 | CGATGTATCACGTA | 139 |
| 60 | CTATGCTCGTGACT | 140 |
| 61 | TATGTGAGACTATA | 147 |
| 62 | CTCGTGAGTCAGTA | 148 |
| 63 | TCTGACGCGAGACT | 155 |
| 64 | TAGTCTATGACTGC | 161 |
| 65 | CGCGACTATGCTGT | 163 |
| 66 | GATCGTATGTGCGC | 167 |
| 67 | AGATCATACAGACT | 168 |
| 68 | TATGAGCGTGCTGC | 170 |
| 69 | TCGATATATGCTGC | 177 |
| 70 | AGCACTCTCGTATG | 185 |
| 71 | CTATCATATGCGTA | 187 |
| 72 | TCGTGTCGTGCTGT | 197 |
| 73 | GCTGTAGCGTAGTG | 198 |
| 74 | TCTGTGCGTGTACT | 202 |
| 75 | GAGATATCGTGATA | 209 |
| 76 | TCGACTAGACAGTA | 235 |
| 77 | GCGACATATCAGTG | 239 |
| 78 | CGCACTATGTAGTA | 245 |
| 79 | AGCGACGACAGCGT | 261 |
| 80 | CTCATATCGTGCGC | 263 |
| 81 | GAGACTATGAGATG | 270 |
| 82 | GCTCGCTACGTCAG | 295 |
| 83 | CGCACTCGTGCTCA | 299 |
| 84 | GCGTGCTACAGACA | 311 |
| 85 | AGCATGCTCACGAC | 322 |
| 86 | TCGTCAGACACGAC | 325 |
| 87 | AGCGTAGCATAGAC | 330 |
| 88 | TATCAGAGATGCGT | 342 |
| 89 | TCGTCTATCGTCGT | 344 |
| 90 | CTCGACGACACGTA | 360 |
| 91 | AGACACGACGCTCA | 362 |
| 92 | CTATCTCGTCTCAC | 368 |
| 93 | TCTCACTATCATGC | 380 |
| 94 | GCTGAGATCACGAC | 381 |
| 95 | ATATGCGATCATCT | 386 |
| 96 | GCGTGCTCATATCT | 401 |
| 97 | TCTCGTATCGCGTG | 405 |
| 98 | AGATCTCTGTGACA | 414 |
| 99 | GAGATATACGCGAG | 424 |
| 100 | ATCGTGAGATGATG | 437 |
| 101 | TCTCACGCACTATG | 440 |
| 102 | GCGATGCGTGTCAC | 445 |
| 103 | TCGTGTCGATAGAG | 458 |
| 104 | GATCACTATGCGTA | 484 |
| 105 | CTACGTAGTCTATA | 535 |
| 106 | GAGTGCGCACTCGT | 541 |
| 107 | TCGTGCTATGTATG | 542 |
| 108 | AGATGTAGACTACT | 581 |
| 109 | ATACAGAGATATGC | 594 |
| 110 | CTACAGCTCGTATA | 596 |
| 111 | CGACGTCTGAGATG | 608 |
| 112 | CTCGAGCGATGCGC | 625 |
| 113 | TAGATAGATCATCA | 664 |
| 114 | AGCATGCGACTACA | 669 |
| 115 | CGCGAGCTGACTCA | 680 |
| 116 | GATGTAGACGTCGC | 756 |
| 117 | CGCATGATCAGATG | 779 |
| 118 | GCGTCATCGAGCGC | 790 |
| 119 | CTACAGCGTCATCT | 817 |
| 120 | ATACGCGCACTCAC | 893 |
| 121 | CTATGTATGAGCAC | 899 |
| 122 | CTCGTGATGACGAG | 951 |
| 123 | CGCGACGCACATGC | 997 |
| 124 | CTATCAGACGTCAG | 1049 |
| 125 | ATACAGAGTGCGAG | 1112 |
| 126 | ATACGTATCACTGC | 1251 |
| 127 | CGACAGCTCAGCAC | 1311 |
| 128 | CTATCAGCGAGATG | 1355 |
| 129 | ATCACTCGATAGTG | 1376 |
| 130 | CGCATATCATATCT | 1399 |
| 131 | CGCGAGAGTGTACA | 1525 |
| 132 | GAGATGCTGAGCGC | 1568 |
| 133 | AGCATGAGTGCTGT | 1589 |
| 134 | CGCGTAGCGAGCAG | 1778 |
| 135 | AGATCATCATGCAC | 1890 |
| 136 | TCTGTATACACGTA | 1909 |
| 137 | TCTGTATCATGACA | 2169 |
| 138 | TCGTCTCTCAGATA | 2196 |
| 139 | ATACACGATGTATG | 2609 |
| 140 | ATACGCTCGACTCA | 2833 |
| 141 | TCTGACGATGCGAG | 2857 |
| 142 | GATGAGCGTCAGAG | 2910 |
| 143 | GAGTCTCGTCATCT | 3395 |
| 144 | ATACGTAGATGCAG | 3415 |
| 145 | GATCACGATCTCAC | 3428 |
| 146 | ATCACTAGTCATGC | 3651 |
| 147 | TAGTGCGCGTGATG | 3680 |
| 148 | GCTCAGATCGTACT | 4170 |
| 149 | GCTGACTACACTCT | 4243 |
| 150 | AGCGAGAGACATCT | 4391 |
| 151 | TCTCGTCTGACTCT | 4568 |
| 152 | GATGACGCACAGTA | 5440 |
| 153 | ATCACAGATGCGAC | 5554 |
| 154 | GCGACTCTCGCTGC | 5938 |
| 155 | CGACAGCGATGACA | 7003 |
| 156 | AGATCAGCACATCA | 7229 |
| 157 | GCTGTGAGATGCAC | 7364 |
| 158 | GCGATAGCATGCAG | 8343 |
| 159 | GATCGTAGTGCGAC | 8520 |
| 160 | CTCACATACGCTCT | 8522 |
| 161 | CGATGTCGATATGT | 8744 |
| 162 | ATCACTATGACTCT | 8809 |
| 163 | TCGATAGCGTATGT | 8963 |
| 164 | ATACACTCGTAGAG | 9308 |
| 165 | GCGTGTATGTGACT | 9847 |
| 166 | TATCAGATGACGTA | 10090 |
| 167 | GAGACTCTGTAGAC | 10629 |
| 168 | GCGTGCGCGACGTA | 10690 |
| 169 | TAGTCAGATCTCGC | 11570 |
| 170 | AGACAGCGACTCGT | 12132 |
| 171 | CGATCAGATCAGAC | 12906 |
| 172 | GATGACGACAGATG | 13442 |
| 173 | AGCGAGATGTGCAC | 13445 |
| 174 | GCGTCAGCACTATA | 13898 |
| 175 | AGCGTATACACTGC | 14072 |
| 176 | CTCATGCTGTGACT | 15131 |
| 177 | GAGATGATCGTATA | 15693 |
| 178 | TATGACTATCTACT | 16035 |
| 179 | TAGATGATGTAGTG | 17311 |
| 180 | TCGACATCACTCGT | 19265 |
| 181 | GCTGACTCACTCGC | 19811 |
| 182 | GAGTGTCTCGTCAC | 19988 |
| 183 | CGACACTCGAGCGT | 21276 |
| 184 | TATCACGCGACTGC | 21882 |
| 185 | AGATGTATGTAGAG | 21955 |
| 186 | AGACGTCGACAGAC | 22214 |
| 187 | ATCGAGCTCGTCAG | 22328 |
| 188 | TCTGAGATGTATGC | 25668 |
| 189 | AGCGACTCGACGAC | 25671 |
| 190 | GAGACATATGTACT | 31084 |
| 191 | GCTGTGAGTGCGTG | 34906 |
| 192 | CTCATGCGTCTCGT | 34923 |
| 193 | TATGACTCGTGCAC | 42593 |
| 194 | AGACGCTCACATGT | 43050 |
| 195 | CGCGTAGATGCGTG | 48624 |
| 196 | CTACACGACAGACT | 50109 |
| 197 | CGATGTCGTGCGAG | 66603 |
| 198 | TAGATGCGACAGAC | 93912 |

## Patentansprüche

1. Verfahren zur Dekonvolution Nukleinsäure enthaltender Substanzgemische, bei dem
a) aus mehreren Nukleotiden (A, C, G, T/U) nach einem vorgegebenen Algorithmus mehrere sich voneinander unterscheidende Target-Nukleotidsequenzen (TNS) (A₁ - Aₙ, B₁ - Bₙ,.... Zₙ) mit N₀-Nₙ Sequenzpositionen generiert werden, von denen
b) jeweils mindestens eine TNS (A₁ - Aₙ, B₁- Bₙ, ..., Zₙ) jeweils mindestens einer Substanz oder Substanzkombination zugeordnet und mit dieser chemisch gekoppelt wird, und
c) mindestens ein zu analysierendes Substanzgemisch mit mindestens zwei unterschiedlichen darin enthaltenen TNS (A₁ - Aₙ, B₁ - Bₙ, ..., Zₙ) oder TNS-gekoppelten Substanzen bereitgestellt wird, das
d) nach einem Sequenzierungsverfahren sequenziert wird, wobei gleichzeitig sämtliche in dem Substanzgemisch enthaltene TNS (A₁ - Aₙ, B₁- Bₙ, ..., Zₙ) in einem gemeinsamen Sequenzspektrum erfasst werden, wobei
e) die im Sequenzspektrum überlagerten Sequenzen durch Abtasten der Sequenzpostionen N₀-Nₙ nach dem vorgegebenen Algorithmus dekonvuliert und gemäß ihrer Zuordnung identifiziert werden, **dadurch gekennzeichnet, dass** eine Nukleotidbelegung für Nukleotide (A, C, G, T/U) an den Sequenzposition N₀ - Nₙ einer zu bildenden TNS (A₁ - Aₙ, B₁- Bₙ, ..., Zₙ) durch eine nach dem vorgegebenen Algorithmus festgelegte Bedingung vorgegeben wird, die an eine Nukleotidbelegung mindestens einer weiteren Sequenzposition geknüpft ist.

2. Verfahren nach Anspruch 1, **dadurch gekennzeichnet, dass** Sequenzpositionen N₀-Nₙ, die im Sequenzspektrum eine signifikant erhöhte Signalintensität einzelner Nukleotide (A, C, G, T/U) aufweisen, nach dem vorgegebenen Algorithmus abgetastet werden.

3. Verfahren nach einem der vorhergenenden Ansprüche, **dadurch gekennzeichnet, dass** im Sequenzspektrum signifikant erhöhte Signalintensitäten einzelner Nukleotide (A, C, G, T/U) schrittweise subtrahiert werden, bis an jeder Sequenzposition N₀-Nₙ für maximal ein Nukleotid (A, C, G, T/U) die kleinste detektierbare Signalintensität erreicht ist, wobei die dabei erhaltenen Subtraktionsspektren, die jeweils zumindest Sequenzabschnitte oder Sequenzfragmente aufweisen, an den Sequenzpositionen N₀-Nₙ nach dem vorgegebenen Algorithmus abgetastet werden.

4. Verfahren nach einem der vorhergehenden Ansprüche, **dadurch gekennzeichnet, dass** nach dem vorgegebenen Algorithmus für jede Sequenzposition N₀₊₁- Nₙ einer zu bildenden TNS (A₁ - Aₙ, B₁- Bₙ, .... Zₙ), eine auf ein Nukleotid (A, C, G, T/U) einer vorhergehenden Sequenzposition bezogene Beschränkung für jeweils mindestens ein Nukleotid (A, C, G, T/U) vorgegeben wird.

5. Verfahren nach einem der vorhergehenden Ansprüche, **dadurch gekennzeichnet, dass** sich die TNS (A₁ - Aₙ, B₁- Bₙ, ..., Zₙ) an mindestens zwei Sequenzpositionen und/oder durch mindestens eine aus mindestens fünf Sequenzpositionen bestehende Nukleotidfolge voneinander unterscheiden.

6. Verfahren nach einem der vorhergehenden Ansprüche, **dadurch gekennzeichnet, dass** die TNS (A₁ - Aₙ, B₁- Bₙ, ..., Zₙ) mit einer Länge von mindestens fünf Sequenzpositionen gebildet werden.

7. Verfahren nach einem der vorhergehenden Ansprüche, **dadurch gekennzeichnet, dass** TNS (A₁ - Aₙ, B₁ - Bₙ, ..., Zₙ), die einen Sequenzunterschied von mindestens 50 %, bevorzugt 75 %, besonders bevorzugt 90 % aufweisen, jeweils Substanzen zugeordnet werden, die den größ-ten strukturellen und/oder funktionellen Unterschied zueinander aufweisen.

8. Verfahren nach einem der vorhergehenden Ansprüche, **dadurch gekennzeichnet, dass** TNS (A₁ - Aₙ, B₁- Bₙ, ..., Zₙ) gebildet werden, die mindestens einen Sequenzabschnitt aufweisen, der eine Substanzgruppe, Substanzgröße, Geokoordinaten eines Expositionsortes oder ein Datum kodiert.

9. Verfahren nach einem der vorhergehenden Ansprüche, **dadurch gekennzeichnet, dass** die TNS (A₁ - Aₙ, B₁- Bₙ, ..., Zₙ) mindestens einen Sequenzabschnitt aufweisen, anhand dem sie als TNS identifizierbar sind.

10. Verfahren nach einem der vorhergenenden Ansprüche, **dadurch gekennzeichnet, dass** es sich bei den TNS (A₁ - Aₙ, B₁- Bₙ, ..., Zₙ) um einzel- oder doppelsträngige RNA- oder DNA-Moleküle handelt.

11. Verfahren nach einem der vorhergenenden Ansprüche, **dadurch gekennzeichnet, dass** die TNS (A₁ - Aₙ, B₁- Bₙ, ..., Zₙ) und/oder Sequenzabschnitte von TNS (A₁ - Aₙ, B₁- Bₙ, ..., Zₙ) miteinander kombiniert werden.

12. Verfahren nach einem der vorhergehenden Ansprüche, **dadurch gekennzeichnet, dass** die TNS (A₁ - Aₙ, B₁ - Bₙ, ..., Zₙ) mit der ihnen zugeordneten Substanz(en) kovalent gebunden werden.

13. Verfahren nach einem der vorhergenenden Ansprüche, **dadurch gekennzeichnet, dass** mindestens ein Verfahrensschritt zur Selektion von mindestens einer der TNS (A₁ - Aₙ, B₁- Bₙ, ..., Zₙ) und/oder mindestens einer TNS- gekoppelten Substanz durchgeführt wird, wobei ein zu analysierendes Substanzgemisch aus einer flüssigen mobilen Phase und/oder aus einer flüssigen Elution einer stationären Phase bereitgestellt wird.

14. Verfahren nach einem der vorhergenenden Ansprüche, **dadurch gekennzeichnet, dass** das zu analysierende Substanzgemisch unter Verwendung fluoreszenzmarkierter Didesoxy-Nukleotide, mindestens einer Polymerase und mindestens eines Primers, der mit einem Sequenzabschnitt mindestens einer TNS (A₁ - Aₙ, B₁ - Bₙ, ..., Zₙ) komplementär ist, sequenziert wird.

## Claims

1. Method for the deconvolution of substance mixtures comprising nucleic acid, in which
a) a plurality of target nucleotide sequences (TNS) (A₁ - Aₙ, B₁ - Bₙ, ..., Zₙ), which differ from each other, with N₀ - Nₙ sequence positions, are generated from a plurality of nucleotides (A, C, G, T/U), according to a prescribed algorithm, of which
b) respectively at least one TNS (A₁ - Aₙ, B₁ - Bₙ, ..., Zₙ) is assigned respectively to at least one substance or substance combination and coupled chemically with the latter, and
c) at least one substance mixture to be analysed, with at least two different TNS (A₁ - Aₙ, B₁ - Bₙ, ..., Zₙ) or TNS-coupled substances contained therein, is prepared, which substance mixture
d) is sequenced according to a sequencing method, all of the TNS (A₁ - Aₙ, B₁ - Bₙ, ..., Zₙ) contained in the substance mixture being detected in a common sequence spectrum at the same time,
e) the sequences superimposed in the sequence spectrum being deconvoluted by scanning the sequence positions N₀ - Nₙ according to the prescribed algorithm and being identified according to their assignment,
**characterized in that** a nucleotide occupancy for nucleotides (A, C, G, T/U) at the sequence positions N₀ - Nₙ of a TNS (A₁ - Aₙ, B₁ - Bn, ..., Zₙ) to be formed is prescribed by a condition which is established according to the prescribed algorithm and is linked to a nucleotide occupancy of at least one further sequence position.

2. Method according to claim 1, **characterised in that** sequence positions No - Nₙ, which have a significantly increased signal intensity of individual nucleotides (A, C, G, T/U) in the sequence spectrum, are scanned according to the prescribed algorithm.

3. Method according to one of the preceding claims, **characterised in that** significantly increased signal intensities of individual nucleotides (A, C, G, T/U) in the sequence spectrum are subtracted step-wise until the smallest detectable signal intensity is reached at any sequence position N₀ - Nₙ for at most one nucleotide (A, C, G, T/U), the thereby obtained subtraction spectra, which have respectively at least sequence portions or sequence fragments, being scanned at the sequence positions NoNₙ according to the prescribed algorithm.

4. Method according to one of the preceding claims, **characterised in that**, according to the prescribed algorithm, for each sequence position N₀₊₁ - Nₙ of a TNS (A₁ - An, B₁ - Bₙ, ..., Zₙ) to be formed, a restriction related to a nucleotide (A, C, G, T/U) of a preceding sequence position is prescribed for respectively at least one nucleotide (A, C, G, T/U).

5. Method according to one of the preceding claims, **characterised in that** the TNS (A₁ - An, B₁ - Bₙ, ..., Zₙ) differ from each other at at least two sequence positions and/or by at least one nucleotide sequence consisting of at least five sequence positions.

6. Method according to one of the preceding claims, **characterised in that** the TNS (A₁ - An, B₁ - Bₙ, ..., Zₙ) are formed with a length of at least five sequence positions.

7. Method according to one of the preceding claims, **characterised in that** TNS (A₁ - An, B₁ - Bₙ, ..., Zₙ), which have a sequence difference of at least 50%, preferably 75%, particularly preferably 90%, are assigned respectively to substances which have the greatest structural and/or functional difference relative to each other.

8. Method according to one of the preceding claims, **characterised in that** TNS (A₁ - An, B₁ - Bₙ, ..., Zₙ) are formed, which have at least one sequence portion which codes a substance group, substance size, geocoordinates of an exposure location or a date.

9. Method according to one of the preceding claims, **characterised in that** the TNS (A₁ - An, B₁ - Bₙ, ..., Zₙ) have at least one sequence portion, by means of which they can be identified as TNS.

10. Method according to one of the preceding claims, **characterised in that** the TNS (A₁ - An, B₁ - Bₙ, ..., Zₙ) concern single- or doublestranded RNA- or DNA molecules.

11. Method according to one of the preceding claims, **characterised in that** the TNS (A₁- An, B₁ - Bₙ, ..., Zₙ) and/or sequence portions of TNS (A₁ - An, B₁ - Bₙ, ..., Zₙ) are combined with each other.

12. Method according to one of the preceding claims, **characterised in that** the TNS (A₁ - An, B₁ - Bₙ, ..., Zₙ) are bonded covalently to the substance(s) assigned to them.

13. Method according to one of the preceding claims, **characterised in that** at least one method step for selection of at least one of the TNS (A₁ - An, B₁ - Bₙ, ..., Zₙ) and/or at least one TNS-coupled substance is implemented, a substance mixture to be analysed made of a liquid mobile phase and/or a liquid elution of a stationary phase being provided.

14. Method according to one of the preceding claims, **characterised in that** the substance mixture to be analysed is sequenced using fluorescence-marked dideoxynucleotides, at least one polymerase and at least one primer which is complementary to a sequence portion of at least one TNS (A₁ - Aₙ, B₁ - Bₙ, ..., Zₙ).

## Revendications

1. Procédé pour la déconvolution de mélanges de substances contenant un acide nucléique, dans lequel
a) à partir de plusieurs nucléotides (A, C, G, T/U), on génère, selon un algorithme prédéfini, plusieurs séquences nucléotidiques cibles (TNS)(A₁ - A_{n,} B₁ - B_{n,}..., Zₙ) différentes les unes des autres, présentant les positions de séquences N₀-Nₙ, parmi lesquelles
b) au moins une TNS(A₁ - A_{n,} B₁ - B_{n,}..., Zₙ) est affectée à au moins une substance ou à une combinaison de substances et est chimiquement couplée à celle-ci, et
c) au moins un mélange de substances à analyser est mis à disposition, comprenant au moins deux TNS(A₁ - A_{n,} B₁ - B_{n,}..., Zₙ) différentes, qui y sont contenues, ou substances couplées aux TNS, qui
d) est séquencé par un procédé de séquençage, la totalité des TNS(A₁ - A_{n,} B₁-B_{n,}..., Zₙ) contenues dans le mélange de substances étant simultanément détectée dans un spectre de séquences commun, dans lequel
e) les séquences superposées dans le spectre de séquences sont déconvoluées par balayage des positions de séquences N₀-Nₙ conformément à l'algorithme prédéfini, et identifiées en fonction de leur affectation,
**caractérisé en ce qu'**une occupation des nucléotides, pour les nucléotides (A, C, G, T/U), est prédéfinie sur les positions de séquences N₀-Nₙ d'une TNS(A₁ - A_{n,} B₁ - B_{n,}..., Zₙ) à former, par une condition définie en fonction de l'algorithme prédéfini, condition qui est liée à une occupation des nucléotides d'au moins une position de séquence supplémentaire.

2. Procédé selon la revendication 1, **caractérisé en ce que** les positions de séquences N₀-Nₙ, qui dans le spectre de séquences présentent une intensité de signal significativement augmentée de nucléotides individuels (A, C, G, T/U), sont balayées conformément l'algorithme prédéfini.

3. Procédé selon l'une des revendications précédentes, **caractérisé en ce que**, dans le spectre de séquences, des intensités de signal significativement augmentées de nucléotides individuels (A, C, G, T/U) sont soustraites pas-à-pas, jusqu'à ce que, sur chaque position de séquence N₀-Nₙ, on atteigne pour un maximum d'un nucléotide (A, C, G, T/U) l'intensité de signal détectable la plus faible, auquel cas les spectres de soustraction obtenus à cette occasion, dont chacun présente au moins des segments de séquences ou des fragments de séquences, sont balayés sur les positions de séquences N₀-Nₙ conformément à l'algorithme prédéfini.

4. Procédé selon l'une des revendications précédentes, **caractérisé en ce que**, conformément à l'algorithme prédéfini, on prédéfinit pour chaque position de séquence N₀₊₁-Nₙ d'une TNS(A₁ - A_{n,} B₁ - B_{n,}..., Zₙ) à former une restriction, se rapportant à un nucléotide (A, C, G, T/U) d'une position de séquence précédente, pour dans chaque cas au moins un nucléotide (A, C, G, T/U).

5. Procédé selon l'une des revendications précédentes, **caractérisé en ce que** les TNS(A₁ - A_{n,} B₁ - B_{n,}..., Zn) se distinguent les unes des autres sur au moins deux positions de séquences et/ou par au moins une séquence nucléotidique constituée d'au moins cinq positions de séquences.

6. Procédé selon l'une des revendications précédentes, **caractérisé en ce que** les TNS(A₁ - A_{n,} B₁ - B_{n,}..., Zₙ) sont formées avec une longueur d'au moins cinq positions de séquences.

7. Procédé selon l'une des revendications précédentes, **caractérisé en ce que** les TNS(A₁ - A_{n,} B₁ - B_{n,}..., Zₙ) qui présentent une différence de séquence d'au moins 50 %, de préférence de 75 %, d'une manière particulièrement préférée de 90 %, sont chacune affectées à des substances qui présentent les unes par rapport aux autres la différence structurelle et/ou fonctionnelle la plus grande.

8. Procédé selon l'une des revendications précédentes, **caractérisé en ce que** l'on forme des TNS(A₁ - A_{n,} B₁ - B_{n,}..., Zₙ) qui présentent au moins un segment de séquence qui code pour un groupe de substances, une taille de substance, des géo-coordonnées d'un site d'exposition ou une date.

9. Procédé selon l'une des revendications précédentes, **caractérisé en ce que** les TNS(A₁ - A_{n,} B₁ - B_{n,}..., Zₙ) présentent au moins un segment de séquence à l'aide duquel elles peuvent être identifiées comme étant des TNS.

10. Procédé selon l'une des revendications précédentes, **caractérisé en ce que**, pour ce qui concerne les TNS(A₁ - A_{n,} B₁ - B_{n,}..., Zₙ), il s'agit de molécules d'ARN ou d'ADN simple brin ou double brin.

11. Procédé selon l'une des revendications précédentes, **caractérisé en ce que** l'on combine les uns aux autres les TNS(A₁ - A_{n,} B₁ - B_{n,}..., Zₙ) et/ou les segments de séquence de TNS(A₁ - A_{n,} B₁ - B_{n,}...., Zₙ).

12. Procédé selon l'une des revendications précédentes, **caractérisé en ce qu'**on lie par liaison covalente les TNS(A₁ - A_{n,} B₁ - B_{n,}..., Zₙ) à la ou aux substances qui leur sont affectées.

13. Procédé selon l'une des revendications précédentes, **caractérisé en ce qu'**on met en oeuvre au moins une étape de procédé pour la sélection d'au moins l'une des TNS(A₁ - A_{n₁} B₁ - B_{n₁}..., Zₙ) et/ou pour au moins une substance couplée aux TNS, dans lequel on met à disposition un mélange de substances à analyser, à partir d'une phase mobile liquide et/ou d'une élution liquide d'une phase stationnaire.

14. Procédé selon l'une des revendications précédentes, **caractérisé en ce que** le mélange de substances à analyser est séquencé par utilisation de didésoxy-nucléotides marqués par fluorescence, d'au moins une polymérase et d'au moins une amorce, qui est complémentaire d'un segment de séquence ayant au moins une TNS(A₁ - A_{n,} B₁ - B_{n,}..., Zₙ).


### SEQUENCE LISTING

<110> Technische Universität Dresden
<120> Verfahren zur Dekonvolution Nukleinsäure enthaltender Substanzgemische
<130> 1
<150> DE 10 2014 200 446.2
   <151> 2014-01-13
<160> 198
<170> BiSSAP 1.3
<210> 1
   <211> 14
   <212> DNA
   <213> -
<400> 1
   gcgatgagac atgt 14
<210> 2
   <211> 14
   <212> DNA
   <213> -
<400> 2
   atcatatacg tata 14
<210> 3
   <211> 14
   <212> DNA
   <213> -
<400> 3
   tagacatcat agag 14
<210> 4
   <211> 14
   <212> DNA
   <213> -
<400> 4
   tatgtgctcg cgag 14
<210> 5
   <211> 14
   <212> DNA
   <213> -
<400> 5
   agatgctatg tcac 14
<210> 6
   <211> 14
   <212> DNA
   <213> -
<400> 6
   tctcgtagtc tcgt 14
<210> 7
   <211> 14
   <212> DNA
   <213> -
<400> 7
   tcgatgatca ctct 14
<210> 8
   <211> 14
   <212> DNA
   <213> -
<400> 8
   gctcagctgt gcag 14
<210> 9
   <211> 14
   <212> DNA
   <213> -
<400> 9
   ctcgagatcg ctgc 14
<210> 10
   <211> 14
   <212> DNA
   <213> -
<400> 10
   cgcactagat gcgt 14
<210> 11
   <211> 14
   <212> DNA
   <213> -
<400> 11
   gctcgcgcga gcac 14
<210> 12
   <211> 14
   <212> DNA
   <213> -
<400> 12
   agcgtgagtc tcag 14
<210> 13
   <211> 14
   <212> DNA
   <213> -
<400> 13
   ctcgactatc agac 14
<210> 14
   <211> 14
   <212> DNA
   <213> -
<400> 14
   cgatgctcat agta 14
<210> 15
   <211> 14
   <212> DNA
   <213> -
<400> 15
   atcgacgcat gcag 14
<210> 16
   <211> 14
   <212> DNA
   <213> -
<400> 16
   gagtgcgatc agag 14
<210> 17
   <211> 14
   <212> DNA
   <213> -
<400> 17
   ctacactcac taca 14
<210> 18
   <211> 14
   <212> DNA
   <213> -
<400> 18
   tatcgtcgat gata 14
<210> 19
   <211> 14
   <212> DNA
   <213> -
<400> 19
   tctgtatatc tcac 14
<210> 20
   <211> 14
   <212> DNA
   <213> -
<400> 20
   gatcactcgt atca 14
<210> 21
   <211> 14
   <212> DNA
   <213> -
<400> 21
   agcatagcga cgta 14
<210> 22
   <211> 14
   <212> DNA
   <213> -
<400> 22
   tctgagcgat agta 14
<210> 23
   <211> 14
   <212> DNA
   <213> -
<400> 23
   atatctctga cgtg 14
<210> 24
   <211> 14
   <212> DNA
   <213> -
<400> 24
   gagtctagac tcag 14
<210> 25
   <211> 14
   <212> DNA
   <213> -
<400> 25
   gagtgtagtg taca 14
<210> 26
   <211> 14
   <212> DNA
   <213> -
<400> 26
   gctgtgctga gata 14
<210> 27
   <211> 14
   <212> DNA
   <213> -
<400> 27
   ctacgtatgt atct 14
<210> 28
   <211> 14
   <212> DNA
   <213> -
<400> 28
   tatcgcgacg tata 14
<210> 29
   <211> 14
   <212> DNA
   <213> -
<400> 29
   gagactcgtg cgtg 14
<210> 30
   <211> 14
   <212> DNA
   <213> -
<400> 30
   tatcgctcac agac 14
<210> 31
   <211> 14
   <212> DNA
   <213> -
<400> 31
   tatcgctaca gcgt 14
<210> 32
   <211> 14
   <212> DNA
   <213> -
<400> 32
   ctcactctca gcag 14
<210> 33
   <211> 14
   <212> DNA
   <213> -
<400> 33
   gatcagctca ctgt 14
<210> 34
   <211> 14
   <212> DNA
   <213> -
<400> 34
   agcgtgctgt atgt 14
<210> 35
   <211> 14
   <212> DNA
   <213> -
<400> 35
   cgacgcgcga cgag 14
<210> 36
   <211> 14
   <212> DNA
   <213> -
<400> 36
   tcgatagaca gatg 14
<210> 37
   <211> 14
   <212> DNA
   <213> -
<400> 37
   gcgatgctgt atca 14
<210> 38
   <211> 14
   <212> DNA
   <213> -
<400> 38
   ctacgcgatg ctgc 14
<210> 39
   <211> 14
   <212> DNA
   <213> -
<400> 39
   cgcacagcac agtg 14
<210> 40
   <211> 14
   <212> DNA
   <213> -
<400> 40
   gcgtcagatg ctca 14
<210> 41
   <211> 14
   <212> DNA
   <213> -
<400> 41
   tagtctcgat gcgc 14
<210> 42
   <211> 14
   <212> DNA
   <213> -
<400> 42
   atcacagcat gaca 14
<210> 43
   <211> 14
   <212> DNA
   <213> -
<400> 43
   ctatgctaca cgac 14
<210> 44
   <211> 14
   <212> DNA
   <213> -
<400> 44
   cgcacagatg tcgt 14
<210> 45
   <211> 14
   <212> DNA
   <213> -
<400> 45
   agacagatga gact 14
<210> 46
   <211> 14
   <212> DNA
   <213> -
<400> 46
   atatcatcgt atgt 14
<210> 47
   <211> 14
   <212> DNA
   <213> -
<400> 47
   gatgtagcac tact 14
<210> 48
   <211> 14
   <212> DNA
   <213> -
<400> 48
   tatgtatcga ctct 14
<210> 49
   <211> 14
   <212> DNA
   <213> -
<400> 49
   cgacagagac agtg 14
<210> 50
   <211> 14
   <212> DNA
   <213> -
<400> 50
   ctcgtagatc atgt 14
<210> 51
   <211> 14
   <212> DNA
   <213> -
<400> 51
   cgacgtctcg tcgt 14
<210> 52
   <211> 14
   <212> DNA
   <213> -
<400> 52
   tagtgcgacg ctct 14
<210> 53
   <211> 14
   <212> DNA
   <213> -
<400> 53
   gagacagaca ctgt 14
<210> 54
   <211> 14
   <212> DNA
   <213> -
<400> 54
   gcgactcgat gaca 14
<210> 55
   <211> 14
   <212> DNA
   <213> -
<400> 55
   agacacgcgt gata 14
<210> 56
   <211> 14
   <212> DNA
   <213> -
<400> 56
   atcatatcac tcag 14
<210> 57
   <211> 14
   <212> DNA
   <213> -
<400> 57
   gatgtatcat atgc 14
<210> 58
   <211> 14
   <212> DNA
   <213> -
<400> 58
   gctcgtcgtc agta 14
<210> 59
   <211> 14
   <212> DNA
   <213> -
<400> 59
   cgatgtatca cgta 14
<210> 60
   <211> 14
   <212> DNA
   <213> -
<400> 60
   ctatgctcgt gact 14
<210> 61
   <211> 14
   <212> DNA
   <213> -
<400> 61
   tatgtgagac tata 14
<210> 62
   <211> 14
   <212> DNA
   <213> -
<400> 62
   ctcgtgagtc agta 14
<210> 63
   <211> 14
   <212> DNA
   <213> -
<400> 63
   tctgacgcga gact 14
<210> 64
   <211> 14
   <212> DNA
   <213> -
<400> 64
   tagtctatga ctgc 14
<210> 65
   <211> 14
   <212> DNA
   <213> -
<400> 65
   cgcgactatg ctgt 14
<210> 66
   <211> 14
   <212> DNA
   <213> -
<400> 66
   gatcgtatgt gcgc 14
<210> 67
   <211> 14
   <212> DNA
   <213> -
<400> 67
   agatcataca gact 14
<210> 68
   <211> 14
   <212> DNA
   <213> -
<400> 68
   tatgagcgtg ctgc 14
<210> 69
   <211> 14
   <212> DNA
   <213> -
<400> 69
   tcgatatatg ctgc 14
<210> 70
   <211> 14
   <212> DNA
   <213> -
<400> 70
   agcactctcg tatg 14
<210> 71
   <211> 14
   <212> DNA
   <213> -
<400> 71
   ctatcatatg cgta 14
<210> 72
   <211> 14
   <212> DNA
   <213> -
<400> 72
   tcgtgtcgtg ctgt 14
<210> 73
   <211> 14
   <212> DNA
   <213> -
<400> 73
   gctgtagcgt agtg 14
<210> 74
   <211> 14
   <212> DNA
   <213> -
<400> 74
   tctgtgcgtg tact 14
<210> 75
   <211> 14
   <212> DNA
   <213> -
<400> 75
   gagatatcgt gata 14
<210> 76
   <211> 14
   <212> DNA
   <213> -
<400> 76
   tcgactagac agta 14
<210> 77
   <211> 14
   <212> DNA
   <213> -
<400> 77
   gcgacatatc agtg 14
<210> 78
   <211> 14
   <212> DNA
   <213> -
<400> 78
   cgcactatgt agta 14
<210> 79
   <211> 14
   <212> DNA
   <213> -
<400> 79
   agcgacgaca gcgt 14
<210> 80
   <211> 14
   <212> DNA
   <213> -
<400> 80
   ctcatatcgt gcgc 14
<210> 81
   <211> 14
   <212> DNA
   <213> -
<400> 81
   gagactatga gatg 14
<210> 82
   <211> 14
   <212> DNA
   <213> -
<400> 82
   gctcgctacg tcag 14
<210> 83
   <211> 14
   <212> DNA
   <213> -
<400> 83
   cgcactcgtg ctca 14
<210> 84
   <211> 14
   <212> DNA
   <213> -
<400> 84
   gcgtgctaca gaca 14
<210> 85
   <211> 14
   <212> DNA
   <213> -
<400> 85
   agcatgctca cgac 14
<210> 86
   <211> 14
   <212> DNA
   <213> -
<400> 86
   tcgtcagaca cgac 14
<210> 87
   <211> 14
   <212> DNA
   <213> -
<400> 87
   agcgtagcat agac 14
<210> 88
   <211> 14
   <212> DNA
   <213> -
<400> 88
   tatcagagat gcgt 14
<210> 89
   <211> 14
   <212> DNA
   <213> -
<400> 89
   tcgtctatcg tcgt 14
<210> 90
   <211> 14
   <212> DNA
   <213> -
<400> 90
   ctcgacgaca cgta 14
<210> 91
   <211> 14
   <212> DNA
   <213> -
<400> 91
   agacacgacg ctca 14
<210> 92
   <211> 14
   <212> DNA
   <213> -
<400> 92
   ctatctcgtc tcac 14
<210> 93
   <211> 14
   <212> DNA
   <213> -
<400> 93
   tctcactatc atgc 14
<210> 94
   <211> 14
   <212> DNA
   <213> -
<400> 94
   gctgagatca cgac 14
<210> 95
   <211> 14
   <212> DNA
   <213> -
<400> 95
   atatgcgatc atct 14
<210> 96
   <211> 14
   <212> DNA
   <213> -
<400> 96
   gcgtgctcat atct 14
<210> 97
   <211> 14
   <212> DNA
   <213> -
<400> 97
   tctcgtatcg cgtg 14
<210> 98
   <211> 14
   <212> DNA
   <213> -
<400> 98
   agatctctgt gaca 14
<210> 99
   <211> 14
   <212> DNA
   <213> -
<400> 99
   gagatatacg cgag 14
<210> 100
   <211> 14
   <212> DNA
   <213> -
<400> 100
   atcgtgagat gatg 14
<210> 101
   <211> 14
   <212> DNA
   <213> -
<400> 101
   tctcacgcac tatg 14
<210> 102
   <211> 14
   <212> DNA
   <213> -
<400> 102
   gcgatgcgtg tcac 14
<210> 103
   <211> 14
   <212> DNA
   <213> -
<400> 103
   tcgtgtcgat agag 14
<210> 104
   <211> 14
   <212> DNA
   <213> -
<400> 104
   gatcactatg cgta 14
<210> 105
   <211> 14
   <212> DNA
   <213> -
<400> 105
   ctacgtagtc tata 14
<210> 106
   <211> 14
   <212> DNA
   <213> -
<400> 106
   gagtgcgcac tcgt 14
<210> 107
   <211> 14
   <212> DNA
   <213> -
<400> 107
   tcgtgctatg tatg 14
<210> 108
   <211> 14
   <212> DNA
   <213> -
<400> 108
   agatgtagac tact 14
<210> 109
   <211> 14
   <212> DNA
   <213> -
<400> 109
   atacagagat atgc 14
<210> 110
   <211> 14
   <212> DNA
   <213> -
<400> 110
   ctacagctcg tata 14
<210> 111
   <211> 14
   <212> DNA
   <213> -
<400> 111
   cgacgtctga gatg 14
<210> 112
   <211> 14
   <212> DNA
   <213> -
<400> 112
   ctcgagcgat gcgc 14
<210> 113
   <211> 14
   <212> DNA
   <213> -
<400> 113
   tagatagatc atca 14
<210> 114
   <211> 14
   <212> DNA
   <213> -
<400> 114
   agcatgcgac taca 14
<210> 115
   <211> 14
   <212> DNA
   <213> -
<400> 115
   cgcgagctga ctca 14
<210> 116
   <211> 14
   <212> DNA
   <213> -
<400> 116
   gatgtagacg tcgc 14
<210> 117
   <211> 14
   <212> DNA
   <213> -
<400> 117
   cgcatgatca gatg 14
<210> 118
   <211> 14
   <212> DNA
   <213> -
<400> 118
   gcgtcatcga gcgc 14
<210> 119
   <211> 14
   <212> DNA
   <213> -
<400> 119
   ctacagcgtc atct 14
<210> 120
   <211> 14
   <212> DNA
   <213> -
<400> 120
   atacgcgcac tcac 14
<210> 121
   <211> 14
   <212> DNA
   <213> -
<400> 121
   ctatgtatga gcac 14
<210> 122
   <211> 14
   <212> DNA
   <213> -
<400> 122
   ctcgtgatga cgag 14
<210> 123
   <211> 14
   <212> DNA
   <213> -
<400> 123
   cgcgacgcac atgc 14
<210> 124
   <211> 14
   <212> DNA
   <213> -
<400> 124
   ctatcagacg tcag 14
<210> 125
   <211> 14
   <212> DNA
   <213> -
<400> 125
   atacagagtg cgag 14
<210> 126
   <211> 14
   <212> DNA
   <213> -
<400> 126
   atacgtatca ctgc 14
<210> 127
   <211> 14
   <212> DNA
   <213> -
<400> 127
   cgacagctca gcac 14
<210> 128
   <211> 14
   <212> DNA
   <213> -
<400> 128
   ctatcagcga gatg 14
<210> 129
   <211> 14
   <212> DNA
   <213> -
<400> 129
   atcactcgat agtg 14
<210> 130
   <211> 14
   <212> DNA
   <213> -
<400> 130
   cgcatatcat atct 14
<210> 131
   <211> 14
   <212> DNA
   <213> -
<400> 131
   cgcgagagtg taca 14
<210> 132
   <211> 14
   <212> DNA
   <213> -
<400> 132
   gagatgctga gcgc 14
<210> 133
   <211> 14
   <212> DNA
   <213> -
<400> 133
   agcatgagtg ctgt 14
<210> 134
   <211> 14
   <212> DNA
   <213> -
<400> 134
   cgcgtagcga gcag 14
<210> 135
   <211> 14
   <212> DNA
   <213> -
<400> 135
   agatcatcat gcac 14
<210> 136
   <211> 14
   <212> DNA
   <213> -
<400> 136
   tctgtataca cgta 14
<210> 137
   <211> 14
   <212> DNA
   <213> -
<400> 137
   tctgtatcat gaca 14
<210> 138
   <211> 14
   <212> DNA
   <213> -
<400> 138
   tcgtctctca gata 14
<210> 139
   <211> 14
   <212> DNA
   <213> -
<400> 139
   atacacgatg tatg 14
<210> 140
   <211> 14
   <212> DNA
   <213> -
<400> 140
   atacgctcga ctca 14
<210> 141
   <211> 14
   <212> DNA
   <213> -
<400> 141
   tctgacgatg cgag 14
<210> 142
   <211> 14
   <212> DNA
   <213> -
<400> 142
   gatgagcgtc agag 14
<210> 143
   <211> 14
   <212> DNA
   <213> -
<400> 143
   gagtctcgtc atct 14
<210> 144
   <211> 14
   <212> DNA
   <213> -
<400> 144
   atacgtagat gcag 14
<210> 145
   <211> 14
   <212> DNA
   <213> -
<400> 145
   gatcacgatc tcac 14
<210> 146
   <211> 14
   <212> DNA
   <213> -
<400> 146
   atcactagtc atgc 14
<210> 147
   <211> 14
   <212> DNA
   <213> -
<400> 147
   tagtgcgcgt gatg 14
<210> 148
   <211> 14
   <212> DNA
   <213> -
<400> 148
   gctcagatcg tact 14
<210> 149
   <211> 14
   <212> DNA
   <213> -
<400> 149
   gctgactaca ctct 14
<210> 150
   <211> 14
   <212> DNA
   <213> -
<400> 150
   agcgagagac atct 14
<210> 151
   <211> 14
   <212> DNA
   <213> -
<400> 151
   tctcgtctga ctct 14
<210> 152
   <211> 14
   <212> DNA
   <213> -
<400> 152
   gatgacgcac agta 14
<210> 153
   <211> 14
   <212> DNA
   <213> -
<400> 153
   atcacagatg cgac 14
<210> 154
   <211> 14
   <212> DNA
   <213> -
<400> 154
   gcgactctcg ctgc 14
<210> 155
   <211> 14
   <212> DNA
   <213> -
<400> 155
   cgacagcgat gaca 14
<210> 156
   <211> 14
   <212> DNA
   <213> -
<400> 156
   agatcagcac atca 14
<210> 157
   <211> 14
   <212> DNA
   <213> -
<400> 157
   gctgtgagat gcac 14
<210> 158
   <211> 14
   <212> DNA
   <213> -
<400> 158
   gcgatagcat gcag 14
<210> 159
   <211> 14
   <212> DNA
   <213> -
<400> 159
   gatcgtagtg cgac 14
<210> 160
   <211> 14
   <212> DNA
   <213> -
<400> 160
   ctcacatacg ctct 14
<210> 161
   <211> 14
   <212> DNA
   <213> -
<400> 161
   cgatgtcgat atgt 14
<210> 162
   <211> 14
   <212> DNA
   <213> -
<400> 162
   atcactatga ctct 14
<210> 163
   <211> 14
   <212> DNA
   <213> -
<400> 163
   tcgatagcgt atgt 14
<210> 164
   <211> 14
   <212> DNA
   <213> -
<400> 164
   atacactcgt agag 14
<210> 165
   <211> 14
   <212> DNA
   <213> -
<400> 165
   gcgtgtatgt gact 14
<210> 166
   <211> 14
   <212> DNA
   <213> -
<400> 166
   tatcagatga cgta 14
<210> 167
   <211> 14
   <212> DNA
   <213> -
<400> 167
   gagactctgt agac 14
<210> 168
   <211> 14
   <212> DNA
   <213> -
<400> 168
   gcgtgcgcga cgta 14
<210> 169
   <211> 14
   <212> DNA
   <213> -
<400> 169
   tagtcagatc tcgc 14
<210> 170
   <211> 14
   <212> DNA
   <213> -
<400> 170
   agacagcgac tcgt 14
<210> 171
   <211> 14
   <212> DNA
   <213> -
<400> 171
   cgatcagatc agac 14
<210> 172
   <211> 14
   <212> DNA
   <213> -
<400> 172
   gatgacgaca gatg 14
<210> 173
   <211> 14
   <212> DNA
   <213> -
<400> 173
   agcgagatgt gcac 14
<210> 174
   <211> 14
   <212> DNA
   <213> -
<400> 174
   gcgtcagcac tata 14
<210> 175
   <211> 14
   <212> DNA
   <213> -
<400> 175
   agcgtataca ctgc 14
<210> 176
   <211> 14
   <212> DNA
   <213> -
<400> 176
   ctcatgctgt gact 14
<210> 177
   <211> 14
   <212> DNA
   <213> -
<400> 177
   gagatgatcg tata 14
<210> 178
   <211> 14
   <212> DNA
   <213> -
<400> 178
   tatgactatc tact 14
<210> 179
   <211> 14
   <212> DNA
   <213> -
<400> 179
   tagatgatgt agtg 14
<210> 180
   <211> 14
   <212> DNA
   <213> -
<400> 180
   tcgacatcac tcgt 14
<210> 181
   <211> 14
   <212> DNA
   <213> -
<400> 181
   gctgactcac tcgc 14
<210> 182
   <211> 14
   <212> DNA
   <213> -
<400> 182
   gagtgtctcg tcac 14
<210> 183
   <211> 14
   <212> DNA
   <213> -
<400> 183
   cgacactcga gcgt 14
<210> 184
   <211> 14
   <212> DNA
   <213> -
<400> 184
   tatcacgcga ctgc 14
<210> 185
   <211> 14
   <212> DNA
   <213> -
<400> 185
   agatgtatgt agag 14
<210> 186
   <211> 14
   <212> DNA
   <213> -
<400> 186
   agacgtcgac agac 14
<210> 187
   <211> 14
   <212> DNA
   <213> -
<400> 187
   atcgagctcg tcag 14
<210> 188
   <211> 14
   <212> DNA
   <213> -
<400> 188
   tctgagatgt atgc 14
<210> 189
   <211> 14
   <212> DNA
   <213> -
<400> 189
   agcgactcga cgac 14
<210> 190
   <211> 14
   <212> DNA
   <213> -
<400> 190
   gagacatatg tact 14
<210> 191
   <211> 14
   <212> DNA
   <213> -
<400> 191
   gctgtgagtg cgtg 14
<210> 192
   <211> 14
   <212> DNA
   <213> -
<400> 192
   ctcatgcgtc tcgt 14
<210> 193
   <211> 14
   <212> DNA
   <213> -
<400> 193
   tatgactcgt gcac 14
<210> 194
   <211> 14
   <212> DNA
   <213> -
<400> 194
   agacgctcac atgt 14
<210> 195
   <211> 14
   <212> DNA
   <213> -
<400> 195
   cgcgtagatg cgtg 14
<210> 196
   <211> 14
   <212> DNA
   <213> -
<400> 196
   ctacacgaca gact 14
<210> 197
   <211> 14
   <212> DNA
   <213> -
<400> 197
   cgatgtcgtg cgag 14
<210> 198
   <211> 14
   <212> DNA
   <213> -
<400> 198
   tagatgcgac agac 14